# FASCICULE DE BREVET EUROPEEN

(11) **EP 3 706 862 B1**
(45) Date de publication et mention de la délivrance du brevet: **28.07.2021**
(21) Numéro de dépôt: 18804373.1
(22) Date de dépôt: 06.11.2018
(51) Int. Cl.: A61N 2/06, A61N 2/12

(54) **APPAREIL PORTATIF POUR GÉNÉRER UN COURANT ÉLECTRIQUE INDUIT SINUSOÏDAL À BASSE FRÉQUENCE**
TRAGBARES GERÄT ZUR ERZEUGUNG EINES INDUZIERTEN NIEDERFREQUENTEN SINUSFÖRMIGEN ELEKTRISCHEN STROMS
PORTABLE APPARATUS FOR GENERATING AN INDUCED LOW-FREQUENCY SINUSOIDAL ELECTRIC CURRENT

(30) Priorité: 07.11.2017 FR 1760443
(43) Date de publication de la demande: 16.09.2020
(73) Titulaire: G.C. Technology, 13854 Aix-en-Provence (FR)
(72) Inventeur: CREPIN, Gérard, 13090 Aix En Provence (FR); MENEROUD, Patrick, 38450 Vif (FR); RUDENT, Pascal, 13390 Auriol (FR)
(74) Mandataire: Cabinet Beau de Loménie
(86) Numéro de dépôt international: PCT/FR2018/052728
(87) Numéro de publication internationale: WO 2019/092349

(56) Documents cités:
- WO-A1-2008/014902
- DE-A1- 4 132 078
- US-A1- 2014 163 305

## Description

### Arrière-plan de l'invention

La présente invention se rapporte au domaine général de la magnétothérapie, et notamment aux appareils portatifs qui génèrent un courant électrique induit sinusoïdal à basse fréquence destiné à être appliqué sur une zone du corps humain dans le but d'exercer une action antalgique et anti-inflammatoire.

De façon connue, pour générer un courant électrique induit, la magnétothérapie utilise un champ magnétique variable notamment pour soulager les douleurs articulaires et les douleurs péri-articulaires, ce champ magnétique variable étant créé par une source magnétique, une bobine ou un aimant. Dans le cas d'aimants, ces derniers sont mis en mouvement pour créer le courant induit.

La magnétothérapie est généralement mise en œuvre à l'aide d'un appareil (le plus souvent portatif) déplacé au niveau de la zone du corps à traiter, cet appareil renfermant des aimants, par exemple rotatifs, et un moteur pour permettre à ces aimants de générer un champ magnétique variable induisant un champ électrique proportionnel à la vitesse de la variation (loi de Faraday).

On connaît ainsi du document WO 2008/014902 un appareil portatif pour générer un champ magnétique sinusoïdal à des fins thérapeutiques, cet appareil comprenant quatre secteurs angulaires d'aimant qui sont plats, de même forme géométrique, centrés sur un même axe de rotation et espacés angulairement les uns des autres, la polarité de deux secteurs angulaires d'aimant adjacents étant opposée. Ainsi disposés, les secteurs angulaires d'aimant sont entraînés en rotation par un moteur afin de générer un champ magnétique sinusoïdal à une fréquence prédéfinie.

Il est par ailleurs connu que l'effet thérapeutique obtenu est lié aux courants électriques induits par la rotation des secteurs angulaires d'aimant, à condition que l'intensité de ces courants dépasse un certain seuil. Il est également connu que les courants induits sont proportionnels à la vitesse de déplacement linéaire des aimants. Or, pour une vitesse de rotation donnée, la vitesse de déplacement linéaire est plus importante à l'extrémité des secteurs angulaires d'aimant qu'au niveau de leur axe de rotation. De plus, pour obtenir un champ magnétique sinusoïdal avec l'appareil décrit dans le document WO 2008/014902, il est nécessaire d'utiliser des aimants en forme de secteurs angulaires, ce qui diminue davantage le champ magnétique au centre de l'appareil.

Aussi, l'appareil décrit dans le document WO 2008/014902 présente l'inconvénient que l'intensité des courants électriques induits au niveau du centre de l'appareil ne dépasse pas le seuil nécessaire à l'obtention d'un effet thérapeutique. Or, le centre de l'appareil est typiquement la zone la plus sollicitée par l'utilisateur de l'appareil. En effet, de manière naturelle, l'utilisateur a tendance à centrer l'appareil sur la douleur ou la pathologie à traiter.

Un autre inconvénient de l'appareil décrit dans ce document est que l'espace entre les aimants génère un signal en double pic dans une zone proche de la surface de l'appareil, et donc un taux important de distorsion harmonique.

Par ailleurs, les effets thérapeutiques d'un tel appareil étant obtenus pour des fréquences très basses, de préférence inférieure à 10 Hz, il n'est pas souhaitable d'augmenter le champ *V̅ X B̅* en augmentant la vitesse de rotation des aimants. En effet, cette augmentation du champ *V̅ X B̅* aurait pour conséquence de réduire les effets thérapeutiques obtenus.

Un autre appareil portatif pour générer un champ magnétique est connu de US 2014/163305 A1.

### Objet et résumé de l'invention

La présente invention a donc pour but principal de pallier de tels inconvénients en proposant un appareil permettant de générer un courant électrique induit sinusoïdal à basse fréquence dont l'intensité dépasse un seuil prédéfini sur l'ensemble de la surface couverte par l'appareil.

Ce but est atteint grâce à un appareil portatif pour générer un champ magnétique destiné à induire un courant électrique sinusoïdal à basse fréquence dans une zone du corps humain sur laquelle il est appliqué, l'appareil comprenant quatre secteurs angulaires d'aimant de même forme géométrique, centrés sur un même axe de rotation et espacés angulairement les uns des autres, la polarité de deux secteurs angulaires d'aimant adjacents étant opposée, et des moyens de mise en rotation à une vitesse prédéterminée des secteurs angulaires d'aimant autour de l'axe de rotation de façon à générer un courant induit sinusoïdal à une fréquence prédéfinie, appareil dans lequel, conformément à l'invention, chaque secteur angulaire d'aimant comprend une ouverture angulaire intérieure au niveau de l'axe de rotation comprise entre 85 et 90°, une ouverture angulaire extérieure au niveau d'une extrémité libre opposée à l'axe de rotation comprise entre 20° et 50°, et deux bords latéraux définissant un rayon s'étendant sur une distance qui est comprise entre un tiers et deux-tiers d'une distance séparant l'axe de rotation de l'extrémité libre des secteurs angulaires d'aimant.

Les inventeurs ont constaté que la forme particulière des secteurs angulaires d'aimant avec une ouverture angulaire intérieure au niveau de l'axe de rotation comprise entre 85 et 90°, une ouverture angulaire extérieure au niveau d'une extrémité libre opposée à l'axe de rotation comprise entre 20° et 50° (et de préférence égale à 45°), et deux bords latéraux définissant un rayon s'étendant sur une distance qui est comprise entre un tiers et deux-tiers d'une distance séparant l'axe de rotation de l'extrémité libre des secteurs angulaires d'aimant permet d'obtenir un champ magnétique sinusoïdal dont l'intensité est homogène et au-dessus d'un seuil prédéfini entre l'axe de rotation et l'extrémité libre des secteurs angulaires d'aimant.

En particulier, il a été constaté que cet arrangement des secteurs angulaires d'aimant permet d'obtenir un accroissement de l'ordre de 70% de l'intensité du champ magnétique sinusoïdal au niveau du centre de l'appareil par rapport à l'appareil décrit dans le document WO 2008/014902. Il a également été observé que l'inhomogénéité du champ magnétique sur toute la surface de l'appareil est réduite de plus de 35% par rapport à l'appareil du document WO 2008/014902. Enfin, la forme particulière des secteurs angulaires d'aimants permet de réduire le taux de distorsion harmonique (jusqu'à 25% par rapport à la forme des secteurs d'aimants décrits dans le document WO 2008/014902).

Dans un mode de réalisation, la distance séparant l'axe de rotation de l'extrémité libre de chaque secteur angulaire d'aimant est de 60mm, le rayon de jointure définit par les bords latéraux des secteurs angulaires d'aimant étant de 35mm.

Les secteurs angulaires d'aimant peuvent posséder une épaisseur sensiblement constante. Dans ce cas, les secteurs angulaires d'aimant possèdent de préférence une épaisseur comprise entre 2 mm et 15 mm.

Alternativement, les secteurs angulaires d'aimant peuvent posséder une épaisseur variable entre l'axe de rotation et leur extrémité libre. Dans ce cas, l'épaisseur des secteurs angulaires d'aimant est de préférence décroissante depuis l'axe de rotation entre une épaisseur maximale et une épaisseur minimale. De plus, chaque secteur angulaire d'aimant présente une face arrière sensiblement perpendiculaire à l'axe de rotation et une face avant opposée à la face arrière qui est inclinée par rapport à la face arrière.

De préférence également, l'appareil comprend en outre une couche de circuit magnétique disposée au dos des secteurs angulaires d'aimants. Cette couche de circuit magnétique peut être réalisée en acier doux.

La présence de cette couche de circuit magnétique au dos des secteurs angulaires d'aimants permet de réduire la charge de ces derniers et d'augmenter le champ magnétique qu'ils génèrent. De plus, la surface d'aimant en vis-à-vis de la zone de soin est modifiée par augmentation de la proportion relative d'aimant sur les petits rayons (afin d'augmenter la source de champ magnétique sur ces petits rayons) et par réduction de la surface sur les plus grands rayons, ce qui rend le champ magnétique induit plus homogène entre l'axe de rotation et l'extrémité libre des secteurs angulaires d'aimant.

De préférence encore, l'appareil comprend en outre une couche de blindage magnétique disposée autour des secteurs angulaires d'aimant. Cette couche de blindage magnétique, qui peut être réalisée en alliage nickel-fer, permet de limiter fortement le rayonnement du champ magnétique induit en arrière et sur les côtés de l'appareil.

### Brève description des dessins

D'autres caractéristiques et avantages de la présente invention ressortiront de la description faite ci-dessous, en référence aux dessins annexés qui en illustrent un exemple de réalisation dépourvu de tout caractère limitatif. Sur les figures :
- la figure 1 est une vue schématique d'un appareil selon un mode de réalisation de l'invention ;
- la figure 2 est une vue en coupe selon II-II de la figure 1 ;
- la figure 3 montre la distribution du champ *V̅ X B̅* induit obtenu par l'appareil de la figure 1 ;
- la figure 4 montre des courbes d'induction obtenues par l'appareil de la figure 1 sur différents rayons ;
- la figure 5 montre la variation du taux de distorsion harmonique en fonction de l'angle de l'ouverture angulaire extérieure ;
- la figure 6 représente des courbes de comparaison de la distribution de champ par rapport à la distance à l'axe de rotation entre l'appareil selon l'invention et un appareil selon l'art antérieur ;
- la figure 7 est une vue schématique en coupe montrant un appareil selon une variante de réalisation de l'invention.

### Description détaillée de l'invention

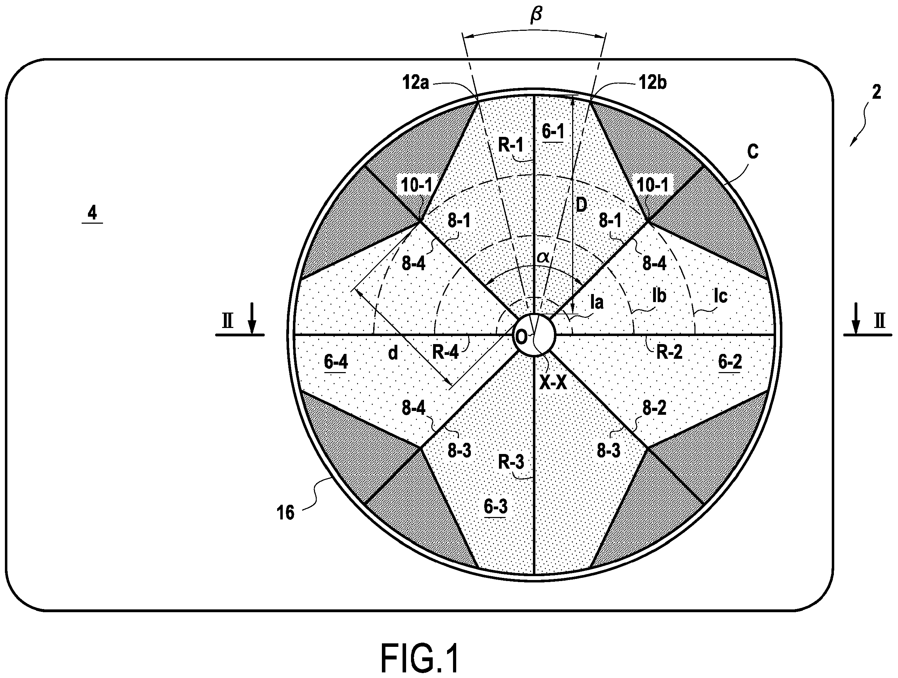

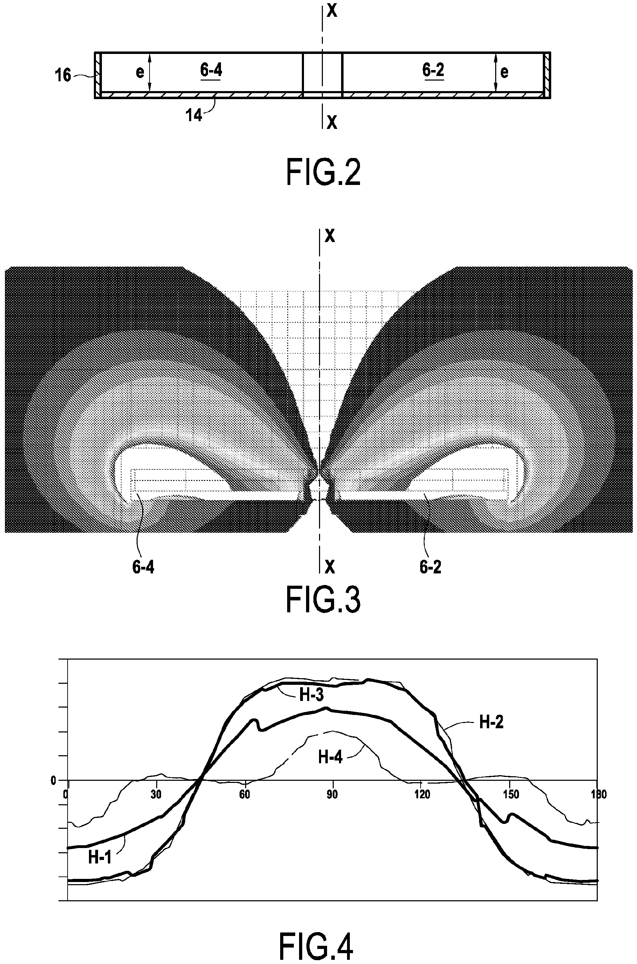

Les figures 1 et 2 représentent de façon schématique un appareil portatif 2 selon l'invention pour générer un courant électrique induit sinusoïdal à basse fréquence destiné à être appliqué sur une zone du corps humain.

Cet appareil 2 comprend un boîtier 4 à l'intérieur duquel sont assemblés quatre secteurs angulaires d'aimant 6-1 à 6-4 ayant une même forme géométrique et centrés sur un même axe de rotation X-X. Ces quatre secteurs angulaires d'aimant 6-1 à 6-4 sont inscrits à l'intérieur d'un cercle C de diamètre D et de centre O.

De façon plus précise, les secteurs angulaires d'aimant 6-1 à 6-4 sont espacés angulairement les uns des autres autour de l'axe de rotation X-X et sont disposés de façon à ce que la polarité (Nord ou Sud) de deux secteurs angulaires d'aimant adjacents soit opposée. En d'autres termes, deux secteurs angulaires d'aimants qui sont diamétralement opposés possèdent la même polarité.

Ainsi, sur l'exemple représenté sur les figures 1 et 2, les secteurs angulaires d'aimant 6-1 et 6-3 ont une polarité N, tandis que les secteurs angulaires d'aimant 6-2 et 6-4 ont une polarité S.

Les secteurs angulaires d'aimant sont typiquement réalisés en aimant terre rare (tels que le Néodyme Fer Bore) dont les caractéristiques sont une aimantation rémanente qui s'étend de 0,83T à 1,47T et une densité d'énergie (BH)max qui s'étend de 135 kJ/m³ à 415 kJ/m³.

L'appareil 2 comprend en outre des moyens pour mettre en rotation les quatre secteurs angulaires d'aimant 6-1 à 6-4 autour de l'axe de rotation X-X.

Dans un exemple de réalisation, ces moyens se présentent sous la forme d'un moteur électrique et d'une transmission par courroie. Bien entendu, tout autre moyen pourrait être envisagé pour assurer cette mise en rotation.

Selon l'invention, les quatre secteurs angulaires d'aimant 6-1 à 6-4 présentent chacun une forme symétrique par rapport à un rayon de symétrie R-1 à R-4, respectivement, du cercle C à l'intérieur duquel ils sont inscrits.

Chaque secteur angulaire d'aimant 6-1 à 6-4 comprend deux bords latéraux 8-1 à 8-4 qui sont symétriques par rapport au rayon de symétrie du secteur angulaire d'aimant et qui sont en regard de ou en contact direct avec des bords latéraux correspondants de deux secteurs angulaires d'aimant adjacents.

De plus, les deux bords latéraux 8-1 à 8-4 de chaque secteur angulaire d'aimant définissent un rayon de jointure (délimité entre le centre O du cercle C et le point 10-1 à 10-4 du bord latéral le plus éloigné du centre O) qui s'étend sur une distance d correspondant à deux-tiers du rayon D/2 du cercle C (c'est-à-dire à deux-tiers de la distance séparant l'axe de rotation X-X de l'extrémité libre des secteurs angulaires d'aimant). Pour des raisons de commodité, seuls les points 10-1 du secteur angulaire d'aimant 6-1 sont représentés sur la figure 1.

Ainsi, dans un exemple de réalisation pour lequel les secteurs angulaires d'aimant sont inscrits dans un cercle C de 120mm de diamètre D, la distance d sur laquelle s'étendent les rayons de jointure définis par les bords latéraux des secteurs angulaires d'aimant est de 35mm.

En outre, les bords latéraux 8-1 à 8-4 de chaque secteur angulaire d'aimant forme entre eux un angle α d'environ 90° (on dit également que l'ouverture angulaire intérieure α de chaque secteur angulaire d'aimant au niveau de l'axe de rotation X-X est comprise entre 85 et 90°).

Toujours selon l'invention, chaque secteur angulaire d'aimant comprend en outre une ouverture angulaire extérieure β au niveau d'une extrémité libre opposée à l'axe de rotation X-X qui est comprise entre 20° et 50°, et de préférence égale à 45°.

En d'autres termes, l'extrémité libre de chaque secteur angulaire d'aimant 6-1 à 6-4 est délimitée entre deux points 12a, 12b situés sur le cercle C dans lequel sont inscrits les secteurs angulaires d'aimant. Ces points sont symétriques et les rayons formés par les points O et 12a, d'une part, et O et 12b, d'autre part, forment entre eux un angle β compris entre 20° et 50°, et de préférence égal à 45°.

Pour des raisons de commodité, seuls les points 12a, 12b et l'angle β de l'ouverture angulaire relative au secteur angulaire d'aimant 6-1 sont représentés sur la figure 1. Bien entendu, les mêmes caractéristiques s'appliquent aux autres secteurs angulaires d'aimant 6-2 à 6-4.

La configuration générale décrite des secteurs angulaires d'aimant de l'appareil selon l'invention présente ainsi une forme d'étoile inscrite dans le cercle C de centre O.

Les secteurs angulaires d'aimant 6-1 à 6-4 de l'appareil selon l'invention sont mis en rotation autour de l'axe de rotation X-X à une vitesse de préférence de 300 tours/minute, ce qui génère un courant électrique induit sinusoïdal à une fréquence de préférence inférieure ou égale à 10Hz.

La figure 3 montre la distribution spatiale du champ *V̅ X B̅* (V étant le champ vitesse et B le champ induction magnétique) induit obtenu par l'appareil selon l'invention lorsque les secteurs angulaires d'aimant sont mis en rotation à une vitesse de 300 tours/minute. Cette distribution est illustrée dans un plan médian aux secteurs angulaires d'aimant.

Cette distribution montre que le champ *V̅ X B̅* induit présente une intensité qui dépasse un seuil prédéfini sur l'ensemble de la surface couverte par l'appareil

Cette figure 3 permet également de montrer que la disposition et la forme particulières des secteurs angulaires d'aimant selon l'invention accroit de manière significative (de l'ordre de 70%) l'intensité du champ magnétique induit proche de l'axe ou sur de faibles rayons par rapport à des secteurs angulaires d'aimant ayant une forme telle que divulguée dans le document WO 2008/014902).

La figure 4 illustre différentes courbes de la composante verticale d'induction obtenues par l'appareil selon l'invention sur différents rayons du cercle dans lequel sont inscrits les secteurs angulaires d'aimant.

Ainsi, la courbe H-1 illustre la composante verticale (c'est à dire selon l'axe de rotation X-X) de l'induction générée au niveau du rayon Ia représenté sur la figure 1, la courbe H-2 illustre celle générée au niveau du rayon Ib, la courbe H-3 illustre celle générée au niveau du rayon Ic, et la courbe H-4 illustre celle générée au niveau rayon D/2 du cercle C. Sur ces courbes, la composante verticale de l'induction est mesurée en microtesla et l'angle du rayon en degrés.

Ces courbes montrent que la distribution de l'induction générée est relativement homogène sur toute la surface de l'appareil. En particulier, l'inhomogénéité du champ magnétique sur toute la surface de l'appareil est réduite de plus de 35% par rapport à l'appareil du document WO 2008/014902.

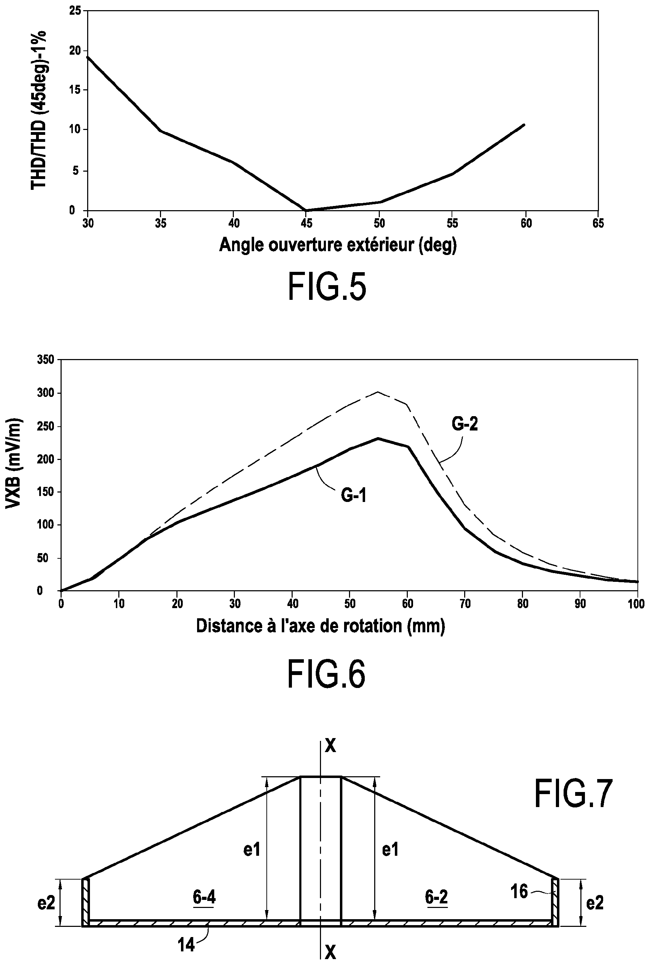

La figure 5 illustre la variation du taux de distorsion harmonique (THD) en fonction de la valeur de l'angle β de l'ouverture angulaire extérieure des secteurs angulaires d'aimant. Cette figure montre que le taux de distorsion harmonique est plus faible pour un angle β de l'ouverture angulaire extérieure égal à 45° environ.

La figure 6 représente des courbes G-1, G-2 de la distribution de champ *V̅ X B̅* (en mV/m) en fonction de la distance à l'axe de rotation X-X pour l'appareil selon l'invention (courbe G-1) et pour un appareil selon l'art antérieur du type décrit dans la publication WO 2008/014902 (courbe G-2). Plus précisément, ces courbes ont été réalisées avec un appareil selon l'invention dans lequel les secteurs angulaires d'aimant ont une épaisseur constante de 4,2mm pour un poids total de 393g, tandis que les secteurs d'aimant de l'appareil selon l'art antérieur ont une épaisseur constante de 10mm pour un poids total de 432g.

On constate sur cette figure qu'une meilleure homogénéité de distribution du champ est obtenue par l'appareil selon l'invention. En particulier, l'appareil selon l'invention permet un gain de 38% entre le maximum et le minimum du champ par rapport à un appareil selon l'art antérieur. De plus, l'appareil selon l'invention permet un gain par rapport à un appareil selon l'art antérieur de 25% du champ à une distance de 5mm de l'axe de rotation des secteurs angulaires d'aimant. Enfin, ces gains sont obtenus avec une réduction de 58% de l'épaisseur des aimants et une baisse de 9% de leur masse totale.

Par ailleurs, dans le mode de réalisation des figures 1 et 2, les secteurs angulaires d'aimant 6-1 à 6-4 possèdent chacun une même épaisseur e qui est sensiblement constante (les secteurs angulaires d'aimant sont plats). De préférence, pour des secteurs angulaires d'aimant inscrits dans un cercle C de 120mm de diamètre, cette épaisseur e est comprise entre 2mm et 10mm.

Dans une variante de réalisation représentée sur la figure 7, les secteurs angulaires d'aimant possèdent chacun une épaisseur qui est variable entre l'axe de rotation X-X et leur extrémité libre.

De façon plus précise, cette épaisseur est décroissante depuis l'axe de rotation avec une épaisseur maximale e1 et une épaisseur minimale e2 l'épaisseur maximale e1 étant comprise entre 2 et 5 fois l'épaisseur minimale e2.

Ainsi, l'épaisseur des secteurs angulaires d'aimant est décroissante depuis l'axe de rotation entre l'épaisseur maximale e1 et l'épaisseur minimale e2. Autrement dit, chaque secteur angulaire d'aimant présente une face arrière (c'est-à-dire opposée à la zone de soin) sensiblement perpendiculaire à l'axe de rotation X-X et une face avant (tournée vers la zone de soin) opposée à la face arrière qui est inclinée par rapport à la face arrière.

Par rapport à une forme plate, la forme biseautée des secteurs angulaires d'aimant permet, d'une part d'accroître davantage l'intensité du champ magnétique induit au niveau du centre de l'appareil, et d'autre de réduire davantage le taux de distorsion harmonique.

On décrira à présent différentes caractéristiques avantageuses de l'appareil selon l'invention qui s'appliquent aux deux modes de réalisation précédemment décrits.

Comme représenté sur la figure 2, l'appareil comprend de façon avantageuse une couche de circuit magnétique 14 qui est disposée au dos des secteurs angulaires d'aimants 6-1 à 6-4 (c'est-à-dire du côté de la face des secteurs angulaires d'aimants qui est opposée à celle tournée vers la zone de soin à traiter).

Par exemple, la couche de circuit magnétique est une couche en acier doux d'épaisseur constante qui recouvre l'ensemble de la surface des secteurs angulaires d'aimants.

Il a été constaté que la présence de cette couche de circuit magnétique permet de réduire la charge des secteurs angulaires d'aimants et d'augmenter le champ magnétique qu'ils génèrent. De plus, la surface d'aimant en vis-à-vis de la zone de soin est modifiée par augmentation de la proportion relative d'aimant sur les petits rayons (afin d'augmenter la source de champ magnétique sur ces petits rayons) et par réduction de la surface sur les plus grands rayons, ce qui rend le champ magnétique induit plus homogène entre l'axe de rotation et l'extrémité libre des secteurs angulaires d'aimant.

Selon une autre disposition avantageuse représentée sur la figure 1, l'appareil comprend en outre une couche de blindage magnétique 16 qui est disposée autour des secteurs angulaires d'aimant (c'est-à-dire selon la circonférence du cercle C dans lequel sont ces derniers sont inscrits). Par exemple, la couche de blindage magnétique d'une épaisseur comprise entre 0,1mm et 2mm est réalisée en mu-métal (c'est-à-dire en alliage de nickel et de fer).

Il a été constaté que la présence de cette couche de blindage permet de limiter fortement le rayonnement du champ magnétique induit en arrière et sur les côtés de l'appareil. On notera que les dimensions des secteurs angulaires d'aimant peuvent être différentes selon l'application retenue. Par exemple, pour obtenir une plus grande profondeur de pénétration du champ magnétique sinusoïdal obtenu, le cercle dans lequel les secteurs angulaires d'aimant sont inscrits pourra avoir un diamètre D compris entre 240 et 300mm. Par exemple encore, pour obtenir un champ magnétique sinusoïdal plus localisé, le cercle dans lequel les secteurs angulaires d'aimant sont inscrits pourra avoir un diamètre D d'environ 20mm.

## Revendications

1. Appareil portatif pour générer un champ magnétique destiné à induire un courant électrique sinusoïdal à basse fréquence dans une zone du corps humain sur laquelle il est appliqué, l'appareil comprenant :
quatre secteurs angulaires d'aimant (6-1 à 6-4) inscrits dans un même cercle (C), centrés sur un même axe de rotation (X-X) et espacés angulairement les uns des autres, la polarité de deux secteurs angulaires d'aimant adjacents étant opposée ; et
des moyens de mise en rotation à une vitesse prédéterminée des secteurs angulaires d'aimant autour de l'axe de rotation de façon à générer un courant induit sinusoïdal à une fréquence prédéfinie ; chaque secteur angulaire d'aimant comprenant une même forme géométrique avec une ouverture angulaire intérieure (α) au niveau de l'axe de rotation comprise entre 85 et 90°, **caractérisé en ce que** chaque secteur angulaire comprend de plus une ouverture angulaire extérieure (β) au niveau d'une extrémité libre opposée à l'axe de rotation comprise entre 20° et 50°, et deux bords latéraux (8-1 à 8-4) définissant un rayon s'étendant sur une distance (d) qui est comprise entre un tiers et deux-tiers d'une distance (D/2) séparant l'axe de rotation de l'extrémité libre des secteurs angulaires d'aimant.

2. Appareil selon la revendication 1, dans lequel l'ouverture angulaire extérieure (β) de chaque secteur angulaire d'aimant (6-1 à 6-4) est de 45°.

3. Appareil selon l'une des revendications 1 et 2, dans lequel la distance (D/2) séparant l'axe de rotation de l'extrémité libre de chaque secteur angulaire d'aimant (6-1 à 6-4) est de 60mm, le rayon de jointure définit par les bords latéraux des secteurs angulaires d'aimant s'étendant sur une distance (d) de 35mm.

4. Appareil selon l'une quelconque des revendications 1 à 3, dans lequel les secteurs angulaires d'aimant (6-1 à 6-4) possèdent une épaisseur (e) sensiblement constante.

5. Appareil selon la revendication 4, dans lequel les secteurs angulaires d'aimant (6-1 à 6-4) possèdent une épaisseur (e) comprise entre 2mm et 15mm.

6. Appareil selon l'une quelconque des revendications 1 à 3, dans lequel les secteurs angulaires d'aimant (6-1 à 6-4) possèdent une épaisseur (e1, e2) variable entre l'axe de rotation et leur extrémité libre.

7. Appareil selon la revendication 6, dans lequel l'épaisseur des secteurs angulaires d'aimant est décroissante depuis l'axe de rotation entre une épaisseur maximale (e1) et une épaisseur minimale (e2).

8. Appareil selon la revendication 7, dans lequel chaque secteur angulaire d'aimant présente une face arrière sensiblement perpendiculaire à l'axe de rotation (X-X) et une face avant opposée à la face arrière qui est inclinée par rapport à la face arrière.

9. Appareil selon l'une quelconque des revendications 1 à 8, comprenant en outre une couche de circuit magnétique (14) disposée au dos des secteurs angulaires d'aimants (6-1 à 6-4).

10. Appareil selon la revendication 9, dans lequel la couche de circuit magnétique (14) est réalisée en acier doux.

11. Appareil selon l'une quelconque des revendications 1 à 10, comprenant en outre une couche de blindage magnétique (16) disposée autour des secteurs angulaires d'aimant (6-1 à 6-4).

12. Appareil selon la revendication 11, dans lequel la couche de blindage magnétique est réalisée en alliage nickel-fer.

## Patentansprüche

1. Tragbares Gerät zur Erzeugung eines Magnetfelds, das dazu bestimmt ist, einen niederfrequenten sinusförmigen elektrischen Strom in einer Zone des menschlichen Körpers, an der es angewandt wird, zu induzieren, wobei das Gerät umfasst:
vier Magnetwinkelsektoren (6-1 bis 6-4), die in einen selben Kreis (C) eingeschrieben sind, auf eine selbe Drehachse (X-X) zentriert sind und winklig voneinander beabstandet sind, wobei die Polarität von zwei benachbarten Magnetwinkelsektoren entgegengesetzt ist, und
Mittel, um Magnetwinkelsektoren mit einer vorbestimmten Drehzahl um die Drehachse herum in Drehung zu versetzen, derart, dass ein induzierter sinusförmiger Strom mit einer vordefinierten Frequenz erzeugt wird,
wobei jeder Magnetwinkelsektor eine gleiche geometrische Form mit einer inneren Winkelöffnung (α) im Bereich der Drehachse von zwischen 85 und 90° umfasst, **dadurch gekennzeichnet, dass** jeder Winkelsektor ferner eine äußere Winkelöffnung (β) im Bereich eines der Drehachse entgegengesetzten freien Endes von zwischen 20° und 50° und zwei seitliche Ränder (8-1 bis 8-4) umfasst, die einen Radius definieren, der sich über eine Distanz (d) erstreckt, die zwischen einem Drittel und zwei Dritteln einer Distanz (D/2) liegt, die die Drehachse von dem freien Ende der Magnetwinkelsektoren trennt.

2. Gerät nach Anspruch 1, wobei die äußere Winkelöffnung (β) von jedem Magnetwinkelsektor (6-1 bis 6-4) 45° beträgt.

3. Gerät nach einem der Ansprüche 1 und 2, wobei die Distanz (D/2), die die Drehachse von dem freien Ende von jedem Magnetwinkelsektor (6-1 bis 6-4) trennt, 60 mm beträgt, wobei der Verbindungsradius, der durch die seitlichen Ränder der Magnetwinkelsektoren definiert ist, sich über eine Distanz (d) von 35 mm erstreckt.

4. Gerät nach einem der Ansprüche 1 bis 3, wobei die Magnetwinkelsektoren (6-1 bis 6-4) eine im Wesentlichen konstante Dicke (e) besitzen.

5. Gerät nach Anspruch 4, wobei die Magnetwinkelsektoren (6-1 bis 6-4) eine Dicke (e) von zwischen 2 mm und 15 mm besitzen.

6. Gerät nach einem der Ansprüche 1 bis 3, wobei die Magnetwinkelsektoren (6-1 bis 6-4) zwischen der Drehachse und ihrem freien Ende eine variable Dicke (e1, e2) besitzen.

7. Gerät nach Anspruch 6, wobei die Dicke der Magnetwinkelsektoren ausgehend von der Drehachse zwischen einer Höchstdicke (e1) und einer Mindestdicke (e2) abnimmt.

8. Gerät nach Anspruch 7, wobei jeder Magnetwinkelsektor eine Rückseite, die im Wesentlichen senkrecht zur Drehachse (X-X) ist, und eine der Rückseite entgegengesetzte Vorderseite aufweist, die in Bezug auf die Rückseite geneigt ist.

9. Gerät nach einem der Ansprüche 1 bis 8, das ferner eine Magnetkreisschicht (14) umfasst, die am Rücken der Magnetwinkelsektoren (6-1 bis 6-4) angeordnet ist.

10. Gerät nach Anspruch 9, wobei die Magnetkreisschicht (14) aus Weichstahl hergestellt ist.

11. Gerät nach einem der Ansprüche 1 bis 10, das ferner eine Magnetabschirmungsschicht (16) umfasst, die um die Magnetwinkelsektoren (6-1 bis 6-4) herum angeordnet ist.

12. Gerät nach Anspruch 11, wobei die Magnetabschirmungsschicht aus einer Nickel-Eisen-Legierung hergestellt ist.

## Claims

1. A portable apparatus for generating a magnetic field intended to induce a low-frequency sinusoidal electric current in an area of the human body to which it is applied, the apparatus comprising:
four angular magnet sectors (6-1 to 6-4) contained in one and the same circle (C), centered on one and the same axis of rotation (X-X) and angularly spaced apart from one another, the polarity of two adjacent angular magnet sectors being opposite; and
means for setting in rotation at a predetermined speed the angular magnet sectors about the axis of rotation such as to generate an induced sinusoidal current at a predefined frequency;
each angular magnet sector comprising one and the same geometrical shape with an internal angular opening (α) at the level of the axis of rotation comprised between 85 and 90°,
**characterized in that** each angular magnet sector further comprises an external angular opening (β) at the level of a free end opposite the axis of rotation comprised between 20° and 50°, and two lateral edges (8-1 to 8-4) defining a radius extending over a distance (d) which is between one-third and two-thirds of a distance (D/2) separating the axis of rotation from the free end of the angular magnet sectors.

2. The apparatus according to claim 1, wherein the external angular opening (β) of each angular magnet sector (6-1 to 6-4) is 45°.

3. The apparatus according to one of claims 1 and 2, wherein the distance (D/2) separating the axis of rotation from the free end of each angular magnet sector (6-1 to 6-4) is 60mm, the join radius defined by the lateral edges of the angular magnet sectors extending over a distance (d) of 35mm.

4. The apparatus according to any one of claims 1 to 3, wherein the angular magnet sectors (6-1 to 6-4) possess a substantially constant thickness (e).

5. The apparatus according to claim 4, wherein the angular magnet sectors (6-1 to 6-4) possess a thickness (e) comprised between 2 mm and 15 mm.

6. The apparatus according to any one of claims 1 to 3, wherein the angular magnet sectors (6-1 à 6-4) possess a thickness (e1, e2) that is variable between the axis of rotation and their free end.

7. The apparatus according to claim 6, wherein the thickness of the angular magnet sectors is decreasing from the axis of rotation between a maximum thickness (e1) and a minimum thickness (e2).

8. The apparatus according to claim 7, wherein each angular magnet sector has a rear face substantially perpendicular to the axis of rotation (X-X) and a front face opposite the rear face which is inclined with respect to the rear face.

9. The apparatus according to any one of claims 1 to 8, further comprising a magnetic circuit layer (14) disposed on the back of the angular magnet sectors (6-1 to 6-4).

10. The apparatus according to claim 9, wherein the magnetic circuit layer (14) is made of mild-steel.

11. The apparatus according to any one of claims 1 to 10, further comprising a magnetic shielding layer (16) disposed about the angular magnet sectors (6-1 to 6-4).

12. The apparatus according to claim 11, wherein the magnetic shielding layer is made of nickel-iron alloy.
